(19) Europäisches Patentamt
European Patent Office
Office européen des brevets

(11) Publication number: **0 137 539**
**B1**

# (12) EUROPEAN PATENT SPECIFICATION

(45) Date of publication of patent specification: **04.05.88**

(51) Int. Cl.[4]: **C 07 D 279/06,** A 01 N 43/86

(21) Application number: **84201270.0**

(22) Date of filing: **04.09.84**

## (54) N-substituted tetrahydrothiazines, process for their preparation, and their use as pesticides.

(30) Priority: **14.09.83 GB 8324664**

(43) Date of publication of application:
**17.04.85 Bulletin 85/16**

(45) Publication of the grant of the patent:
**04.05.88 Bulletin 88/18**

(84) Designated Contracting States:
**AT BE CH DE FR GB IT LI LU NL SE**

(56) References cited:
**GB-A-1 513 951**
**GB-A-2 118 177**
**US-A-3 933 809**
**US-A-4 052 388**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Harris, Martin**
**54 Northwood Drive**
**Sittingbourne Kent (GB)**

(74) Representative: **Hunter, Keith Roger Ian et al**
**4 York Road**
**London SE1 7NA (GB)**

**EP 0 137 539 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to N-substituted nitromethylenetetrahydrothiazines, to processes for their preparation and to their use as pesticides, in particular against insect pests.

Certain 2-nitromethylene-tetrahydro-2H-1,3-thiazines and their use as pesticides are known from GB—B—1,513,951 and GB—A—2,118,177. However, although these thiazines possess a high level of insecticidal activity they suffer from the disadvantage of being unstable to light and, thus, show little persistence under field conditions. The Applicant has now found that this light-stability problem can be overcome by employing a new class of insecticidally-active thiazines.

Accordingly the invention provides an N-substituted 2-nitromethylene-tetrahydro-2H-1,3-thiazine of the general formula:

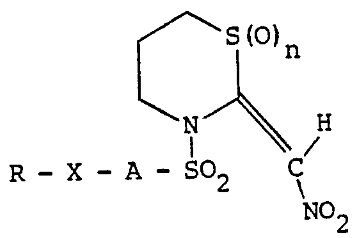
(I)

in which n represents 0 or 1; A represents an alkylene group having from 1 to 10 carbon atoms; X represents an oxygen or sulphur atom or a group —$NR^1$— in which $R^1$ represents a hydrogen atom or an alkyl group having up to 6 carbon atoms, or, together with R, represents an alkylene group having 4 to 6 carbon atoms; and R represents a hydrogen atom; an alkyl, haloalkyl, alkoxyalkyl, tetrahydropyranyl, alkylcarbamoyl, dialkylcarbamoyl, formyl, alkanoyl or haloalkanoyl group, in which any alkyl moiety has up to 6 carbon atoms; a cycloalkanoyl group having 3 to 6 ring carbon atoms optionally substituted by one or more halogen atoms and/or alkyl groups having up to 6 carbon atoms, haloalkyl groups having up to 6 carbon atoms, or dichlorovinyl groups; or a phenyl or benzoyl group optionally substituted by substituents selected from halogen atoms and alkyl, alkoxy, haloalkyl, haloalkoxy, cyano, nitro, amino, hydroxy, dimethylaminophenylazo and alkoxycarbonyl groups, in which any alkyl moiety has up to 6 carbon atoms; provided that if X is oxygen then R is not alkyl, alkanoyl or optionally substituted phenyl.

Throughout this Specification and claims, unless otherwise stated, any alkyl moiety has up to 6, especially up to 4, carbon atoms.

The alkylene group A may be straight-chain or branched and has from 1 to 10, especially 2 to 6, carbon atoms. Preferably A is a straight-chain alkylene group having 3, 4 or 5 carbon atoms.

If $R^1$ together with R represents an alkylene group, this group has 4 to 6 carbon atoms, for example a tetramethylene or pentamethylene group. Preferably $R^1$ is a C(1—4) alkyl, especially methyl, group, or a hydrogen atom.

Preferably X represents an oxygen atom.

Preferably n is 0.

If R represents an optionally substituted phenyl or benzoyl group, the optional substituents are selected from halogen atoms and alkyl, alkoxy, haloalkyl, haloalkoxy, cyano, nitro, amino, hydroxy, dimethylaminophenylazo and alkoxycarbonyl groups. A cycloalkanoyl group R has 3 to 6 ring carbon atoms and is preferably unsubstituted or substituted by up to 4 chlorine atoms and/or methyl and dichlorovinyl groups.

Subject to the above proviso, R preferably represents a hydrogen atom, a C(1—6) alkyl group, a C(1—6) haloalkyl group containing up to 3 halogen, especially chlorine or bromine atoms, an alkoxyalkyl group having 1 to 4 carbon atoms in each alkyl moiety, a tetrahydropyranyl group, a 2,2-dimethyl-3-(2,2-dichlorovinyl)cyclopropylcarbonyl group, a formyl group, a C(2—5)alkanoyl group, a C(2—5) haloalkanoyl group containing up to 3 halogen, especially chlorine or bromine, atoms, or an optionally substituted phenyl or benzoyl group in which the optional substituents are selected from halogen atoms and C(1—4) alkyl, trifluoromethyl, cyano, nitro and dimethylaminophenylazo groups.

Especially preferred are compounds of the general formula I in which R represents a hydrogen atom; an alkoxyalkyl group having 1 to 2 carbon atoms in each alkyl moiety; a formyl group; a benzoyl group; or a dimethylaminophenylazobenzoyl group.

It will be appreciated that the compounds of formula I are capable of existing in different geometrically isomeric forms. The invention includes both the individual isomers and mixtures of such isomers.

The invention includes a process for the preparation of the N-substituted 2-(nitromethylene)-tetrahydro-2H-1,3-thiazines of formula I, which comprises reacting the compound of formula:

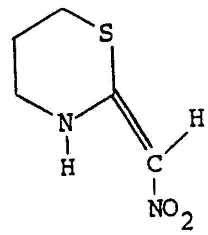

(II)

with a sulphonyl halide of formula

$$R—X—A—SO_2—Hal$$

in which R, X and A are as defined above, and Hal represents a halogen, preferably chlorine, atom, in the presence of a base, to produce a compound of the general formula I in which n is 0; and if desired, converting the resulting compound of the general formula I into any other desired compound of the general formula I.

The base is preferably an organic base such as a tertiary amine, for example a trialkylamine, triethylamine being particularly preferred. The reaction is suitably carried out at a temperature of 0°C or below, for example at a temperature from −75° to −10°C. The reaction is suitably carried out in an organic solvent, for example, a chlorinated hydrocarbon such as dichloromethane, an ether such as tetrahydrofuran, or an amide such as dimethylformamide.

Compounds of the general formula I in which X represents an oxygen or sulphur atom and R represents a formyl, alkanoyl, haloalkanoyl, optionally substituted cycloalkanoyl or optionally substituted benzoyl group may be prepared by an alternative procedure which comprises reacting a compound of the general formula

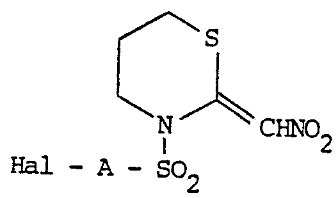

(III)

in which Hal represents a halogen atom and A has the meaning given for the general formula I, with an acid of the general formula

$$R X H$$

or a salt, especially an alkali metal salt, thereof, in which R and X have the meanings given above. Compounds of the general formula III are described and claimed in GB—A—2118177.

In some cases, it may be convenient to prepare certain compounds of the general formula I from other compounds of the general formula I having a different group R. For example, compounds in which R represents a hydrogen atom may be prepared by hydrolysis of a compound in wich R represents a formyl, alkanoyl, haloalkanoyl, optionally substituted cycloalkanoyl or optionally substituted benzoyl group.

The compounds of formula I in which N is 1 may be prepared by oxidising the corresponding derivative in which n is 0. This may be carried out using conventional oxidising agents, for example peracids such as *m*-chloroperbenzoic acid, or potassium hydrogen persulphate. Conveniently the derivative to be oxidised is dissolved in a suitable solvent, for example a chlorinated hydrocarbon solvent such as chloroform or dichloromethane, or a liquid alkanol such as ethanol.

As mentioned above the N-substituted 2-(nitromethylene)tetrahydro-2H-1,3-thiazines of the invention are of interest as pesticides particularly against insect pests. They exhibit activity against such pests as the larval caterpillar or worm forms of insects, for example, of the genus *Spodoptera* and of the genus *Heliothis*. The compounds also exhibit acceptable stability towards light and oxidation.

Accordingly the invention includes pesticidal compositions comprising a substituted 2-(nitromethylene)-tetrahydro-2H-1,3-thiazine of the invention together with a carrier.

Such a composition may contain a single compound or a mixture of several compounds of the invention. It is also envisaged that different isomers or mixture of isomers may have different levels or spectra of activity and thus compositions may comprise individual isomers or mixtures of isomers. The invention further provides a method of combating pests, particular insect pests, at a locus, which comprises applying to the locus a pesticidally effective amount of compound or composition according to the present invention.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material

which is normally gaseous but which has been compressed to form a liquid, and any of the carriers normally used in formulating pesticidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; butumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hyrocarbons, for example benzene, toluene, and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrate form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters or glycerol, sorbitan, sucrose or pentaerythritol; condensates of these with ethylene oxide and/or propylene oxide; condensation products of fatty alcohol or alkyl phenols, for example *p*-octylphenol or *p*-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates emulsions, suspension concentrates and aerosols. Wettable powders usually contain 25, 50 or 75% w of active ingredient and usually contain in addition to solid inert carrier, 3—10% w of a dispersing agent and, where necessary, 0—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676—0.152 mm), and may be manufactured by agglomeration or impregnation techniques. Generally, granules will contain ½-75% w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% of suspending agents such as protective colloids and thixotropic agents, 0—10% w of other additives such as defoamers, corrosion inhibitors, stabilisers, penetrants and stickers, and water or an organic liquid in which the active ingedient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The compositions of the invention may also contain other ingredients, for example other compounds possessing pesticidal properties.

The thermal stability of the compounds and compositions of the invention may often be improved by the addition of stabilizing amounts, usually 10—100%w based on the active compound, of certain organo nitrogen compounds such as urea, dialkylureas, thiourea or guanidine salts or alkali metal salts of weak acids such as bicarbonates, acetates or benzoates.

The invention is illustrated further in the following Examples.

Example 1A

Preparation of Intermediate, 3-(3-chloropropylsulphonyl)-2-nitromethylene-tetrahydro-2H-1,3-thiazine

To 2-(nitromethylene)tetrahydro-2H-1,3-thiazines (4.8 g) in dichloromethane (100 ml) at −15°C under nitrogen was added triethylamine (5 ml). 3-Chloropropyl sulphonyl chloride (5.9 g) in dichloromethane (50 ml) was then added dropwise over 25 minutes. The mixture was allowed to warm to room temperature, stirred for 1 hour, then washed twice with 2% aqueous HCl. The organic phase was dried over $MgSO_4$ and removed under reduced pressure to yield a solid which was triturated with methanol to give 892 mg of the pure product, melting point 98—99°C.

Example 1B

Preparation of Intermediate, 3-(3-iodopropylsulphonyl)-2-nitromethylene-tetrahydro-2H-1,3-thiazine

The compound of Example 1A (10 g) in acetone (700 ml) was heated under reflux for 16 hours with sodium iodide (17.5 g). The solvent was then removed under reduced pressure and the residue was partitioned between water and dichloromethane. The organic phase was dried ($MgSO_4$) and the solvent was then removed under reduced pressure. The residue was recrystallised from dichloromethane to yield the required product as a yellow crystalline solid melting point 106—109°C.

| Elemental Analysis: | C | H | N |
|---|---|---|---|
| Calculated: | 24.5%; | 3.3%; | 7.1% |
| Found: | 25.3%; | 3.4%; | 7.2% |

Example 2

3-(3-formyloxypropylsulphonyl)-2-nitromethylene-2H-1,3-thiazine

4 g of the compound of Example 1B was added to sodium formate (4 g) in hexamethylphosphoramide (20 ml) at 0°C under nitrogen, and the mixture was then stirred at room temperature for 18 hours. It was then poured into diethyl ether (600 ml), and washed with brine. The organic phase was dried and the solvent evaporated under reduced pressure to give 2.9 g of crystals of the desired product, melting point 89°C.

| Elemental Analysis: | C | H | N |
|---|---|---|---|
| Calculated: | 34.8 | 4.5 | 9.0 |
| Found: | 34.5 | 4.5 | 8.9 |

Example 3

3-(3-[4-(4-dimethylaminophenylazo)benzoyloxy]propylsulphonyl)-2-nitromethylene-tetrahydro-2H-1,3-thiazine

4-(4-dimethylaminophenylazo)benzoic acid (1.35 g) dissolved in hexamethylenephosphoramide (30 ml) was added to sodium hydride (230 mg) in hexamethylenephosphoramide (5 ml) at 0°C under nitrogen. The mixture was stirred for 1 hour and then the compound of Example 1B (2g) was added. The resulting deep red solution was stirred for two and a quarter hours, then poured into diethyl ether (500 ml) and washed with water. The ether layer was dried and evaporated under reduced pressure and the resulting solid was combined with that which precipitated in the separating funnel during the ether/water partition. The combined solids were taken up in hot dichloromethane and sufficent hexane was added to initiate precipitation. The mixture was then cooled and the product filtered off. The yield of product, melting point 163—165°C, was 59%

| Elemental Analysis: | C | H | N |
|---|---|---|---|
| Calculated: | 51.8 | 5.1 | 13.1 |
| Found: | 50.2 | 5.0 | 12.3 |

Example 4

3-(3-hydroxypropylsulphonyl)-2-nitromethylenetetrahydro-2H-1,3-thiazine

2 g of the compound of Example 2 were eluted through a column of netural alumina (200 g) using dichloromethane: methanol 19.1 as eluent. The fractions containing the product were evaporated under reduced pressure and triturated with diethyl ether to yield the desired product, 1.3 g, melting point 80—83°C.

| Elemental Analysis: | C | H | N |
|---|---|---|---|
| Calculated: | 34.0 | 5.0 | 9.9 |
| Found: | 33.9 | 4.6 | 9.3 |

Example 5

3-(3-[2-tetrahydropyranyloxy]propylsulphonyl)-2-nitromethylene-tetrahydro-2H-1,3-thiazine

A mixture of the compound of Example 4 (640 mg), dihydropyran (485 mg), *p*-toluenesulphonic acid (6 mg) and dichloromethane (40 ml) was stirred at room temperature for 20 hours. After washing with

aqueous sodium bicarbonate solution, the organic phase was dried over $MgSO_4$, and the solvent removed by evaporation under reduced pressure to give 1 g of the desired product, melting point 63—64°C.

| Elemental Analysis: | C | H | N |
|---|---|---|---|
| Calculated: | 42.6 | 6.0 | 7.7 |
| Found: | 41.7 | 5.8 | 7.7 |

Example 6

3-(3-[(1-ethoxy)ethoxy]propylsulphonyl)-2-nitromethylene-tetrahydro-2H-1,3-thiazine

*p*-Toluenesulphonic acid (5 mg) was added to a mixture of the compound of Example 4 (400 mg) and ethylvinylether (260 mg) in dichloromethane (10 ml) at 20° and stirred for 1½ hours. The resulting solution was washed with dilute sodium bicarbonate solution, the organic phase was dried over $MgSO_4$, and the solvent evaporated under reduced pressure to give 520 mg of the desired product, melting point 61—63°C.

| Elemental Analysis: | C | H | N |
|---|---|---|---|
| Calculated: | 40.7 | 6.2 | 7.9 |
| Found: | 40.6 | 6.5 | 8.2 |

Examples 7 to 15

By methods analogous to those of the preceding Examples, further compounds of the general formula I were prepared. Details are given in Table 1.

TABLE 1

| Example No. | In the General Formula I A | X | R | n | Melting Point (°C) | Elemental Analysis (%) | C | H | N |
|---|---|---|---|---|---|---|---|---|---|
| 7 | $(CH_2)_4$ | O | CHO | O | 76 | Calc.<br>Found | 37.0<br>37.0 | 4.9<br>4.8 | 8.6<br>8.6 |
| 8 | $(CH_2)_4$ | O | 2,2-dimethyl-3-(2,2-dichlorovinyl) cyclopropylcarbonyl | O | 53 | Calc.<br>Found | 41.9<br>41.7 | 4.9<br>4.9 | 5.7<br>5.5 |
| 9 | $(CH_2)_4$ | O | H | O | 76 | Calc.<br>Found | 36.5<br>36.6 | 5.4<br>5.2 | 9.5<br>9.3 |
| 10 | $(CH_2)_4$ | O | benzoyl | O | 114 | Calc.<br>Found | 48.0<br>48.2 | 5.0<br>5.1 | 7.0<br>7.2 |
| 11 | $(CH_2)_5$ | O | CHO | O | 90 | Calc.<br>Found | 39.0<br>38.8 | 5.3<br>5.2 | 8.3<br>8.2 |
| 12 | $(CH_2)_5$ | O | H | O | 114—115 | Calc.<br>Found | 38.7<br>38.2 | 5.8<br>5.5 | 9.0<br>8.9 |
| 13 | $(CH_2)_3$ | O | $CO.CF_3$ | O | 89—90 | Calc.<br>Found | 31.7<br>31.8 | 5.4<br>5.6 | 7.4<br>7.5 |
| 14 | $(CH_2)_3$ | O | $CO.CHF_2$ | O | 114—115 | Calc.<br>Found | 35.1<br>35.3 | 4.4<br>4.3 | 8.2<br>8.1 |
| 15 | $(CH_2)_3$ | O | $CO.NHCH_3$ | O | | | | | |

Example 13

Pesticidal Activity

The insecticidal activity of compounds of the invention was demonstrated agaisnt the pest *Spodoptera littoralis*, Egyptian cotton leaf-worm. The test method employed appears below.

Solutions or suspensions of the compound were made up over a range of concentrations in 10% acetone/water containing .025% Triton X100 ("Triton" is a trade mark). These solutions were sprayed using a logarithmic spraying machine onto petri dishes containing a nutritious diet on which the *Spodoptera littoralis* larvae had been reared. When the spray deposit had dried each dish was infested with 10 2nd instar larvae. Mortality assessments were made 7 days after spraying.

In each test an $LC_{50}$ for the compound was calculated from the mortality figures and compared with the corresponding $LC_{50}$ for ethyl parathion in the same tests. The results are expressed as

$$\text{toxicity indices} = \frac{LC_{50}\ (\text{parathion})}{LC_{50}\ (\text{test compound})} \times 100$$

and are set out in Table 2 below.

TABLE 2

| Compound of Ex. No. | Toxicity Index against *Spodoptera littoralis after 7 days* |
|---|---|
| 2 | 109 |
| 3 | 260 |
| 4 | 110 |
| 5 | 26 |
| 6 | 57 |
| 7 | 70 |
| 8 | 16 |
| 9 | 65 |
| 10 | 45 |
| 11 | 46 |
| 12 | 83 |
| 13 | 69 |
| 14 | 97 |
| 15 | 109 |

**Claims for the Contracting States: BE CH DE FR GB IT LI LU NL SE**

1. An N-substituted 2-nitromethylene-terahydro-2H-1,3-thiazine of the general formula:

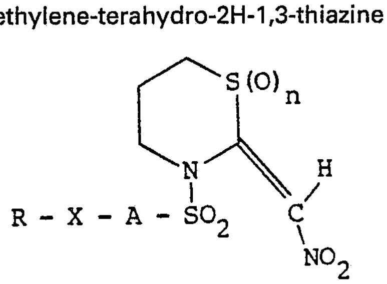

(I)

in which n represents 0 or 1; A represents an alkylene group having from 1 to 10 carbon atoms; X

represents an oxygen or sulphur atom or a group —$NR^1$— in which $R^1$ represents a hydrogen atom or an alkyl group having up to 6 carbon atoms or, together with R, represents an alkylene group having 4 to 6 carbon atoms; and R represents a hydrogen atom; an alkyl, haloalkyl, alkoxyalkyl, tetrahydropyranyl, alkylcarbamoyl, dialkylcarbamoyl, formyl, alkanoyl or haloalkanoyl group, in which any alkyl moiety has up to 6 carbon atoms; a cycloalkanoyl group having 3 to 6 ring carbon atoms optionally substituted by one or more halogen atoms and/or alkyl groups having up to 6 carbon atoms, haloalkyl groups having up to 6 carbon atoms, or dichlorovinyl groups; or a phenyl or benzoyl group optionally substituted by substituents selected from halogen atoms and alkyl, alkoxy, haloalkyl, haloalkoxy, cyano, nitro, amino, hydroxy, dimethylaminophenylazo and alkoxycarbonyl groups, in which any alkyl moiety has up to 6 carbon atoms; provided that if X is oxygen then R is not alkyl, alkanoyl or optionally substituted phenyl.

2. A compound as claimed in claim 1, in which A represents a straight-chain alkylene group having 3, 4 or 5 carbon atoms.

3. A compound as claimed in either claim 1 or claim 2, in which $R^1$ represents a C(1—4) alkyl group or a hydrogen atom.

4. A compound as claimed in either claim 1 or claim 2, in which X represents an oxygen atom.

5. A compound as claimed in any one of claims 1 to 4, in which n is 0.

6. A compound as claimed in any one of claims 1 to 5, in which R represents a hydrogen atom, a C(1—6) alkyl group, a C(1—6) haloalkyl group containing up to 3 halogen atoms, an alkoxyalkyl group having 1 to 4 carbon atoms in each alkyl moiety, a tetrahydropyranyl group, a 2,2-dimethyl-3-(2,2-dichlorovinyl)cyclopropylcarbonyl group, a formyl group, a C(2—5) alkanoyl group, a C(2—5) haloalkanoyl group containing up to 3 halogen atoms, or an optionally substituted phenyl or benzoyl group in which the optional substituents are selected from halogen atoms and C(1—4) alkyl, trifluoromethyl, cyano, nitro and dimethylaminophenylazo groups.

7. A compound as claimed in claim 6, in which R represents a hydrogen atom; an alkoxyalkyl group having 1 or 2 carbon atoms in each alkyl moiety; a formyl group; a benzoyl group; or a dimethylaminophenylazobenzoyl group.

8. A process for the preparation of an N-substituted 2-(nitromethylene)-tetrahydro-2H-1,3-thiazine as claimed in claim 1, which comprises reacting the compound of formula:

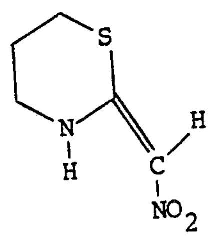

(II)

with a sulphonyl halide of formula

$$R—X—A—SO_2—Hal$$

in which R, X and A are as defined above, and Hal represents a halogen atom, in the presence of a base, to produce a compound of the general formula I in which n is 0; and if desired, converting the resulting compound of the general formula I into any other desired compound of the general formula I.

9. A process for the preparation of a compound as claimed in claim 1 in which X represents an oxygen or sulphur atom and R represents a formyl, alkanoyl, haloalkanoyl, optionally substituted cycloalkanoyl or optionally substituted benzoyl group, which comprises reacting a compound of the general formula

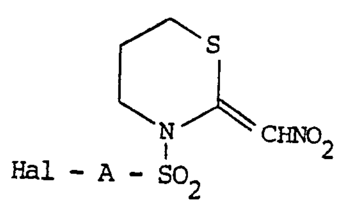

(III)

in which Hal represents a halogen atom and A has the meaning given in claim 1, with an acid of the general formula

$$R\,X\,H$$

or a salt thereof, in which R and X have the meanings given above.

10. A pesticidal composition which comprises a compound as claimed in any one of claims 1 to 7, together with a carrier.

11. A composition as claimed in claim 10, which comprises at least two carriers, at least one of which is a surface-active agent.

12. A method of combating pests at a locus, which comprises applying to the locus a pesticidally effective amount of a compound as claimed in any one of claims 1 to 7 or a composition as claimed in either claim 10 or claim 11.

**Claims for the Contracting State: AT**

1. A pesticidal composition which comprises a carrier and, as active ingredient, an N-substituted 2-nitromethylene-tetrahydro-2H-1,3-thiazine of the general formula:

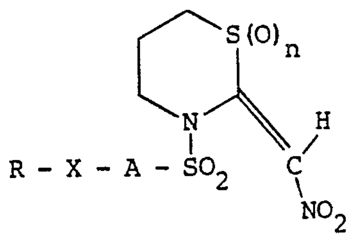

(I)

in which n represents 0 or 1; A represents an alkylene group having from 1 to 10 carbon atoms; X represents an oxygen or sulphur atom or a group —$NR^1$— in which $R^1$ represents a hydrogen atom or an alkyl group having up to 6 carbon atoms or, together with R, represents an alkylene group having 4 to 6 carbon atoms; and R represents a hydrogen atom; an alkyl, haloalkyl, alkoxyalkyl, tetrahydropyranyl, alkylcarbamoyl, dialkylcarbamoyl, formyl, alkanoyl or haloalkanoyl group, in which any alkyl moiety has up to 6 carbon atoms; a cycloalkanoyl group having 3 to 6 ring carbon atoms optionally substituted by one or more halogen atoms and/or alkyl groups having up to 6 carbon atoms, haloalkyl groups having up to 6 carbon atoms, or dichlorovinyl groups; or a phenyl or benzoyl group optionally substituted by substituents selected from halogen atoms and alkyl, alkoxy, haloalkyl, haloalkoxy, cyano, nitro, amino, hydroxy, dimethylaminophenylazo and alkoxycarbonyl groups, in which any alkyl moiety has up to 6 carbon atoms; provided that if X is oxygen then R is not alkyl, alkanoyl or optionally substituted phenyl.

2. A composition as claimed in claim 1, in which A represents a straight-chain alkylene group having 3, 4 or 5 carbon atoms.

3. A composition as claimed in either claim 1 or claim 2, in which $R^1$ represents a C(1—4) alkyl group or a hydrogen atom.

4. A composition as claimed in either claim 1 or claim 2, in which X represents an oxygen atom.

5. A composition as claimed in any one of claims 1 to 4, in which n is 0.

6. A composition as claimed in any one of claims 1 to 5, in which R represents a hydrogen atom, a C(1—6) alkyl group, a C(1—6) haloalkyl group containing up to 3 halogen atoms, an alkoxyalkyl group having 1 to 4 carbon atoms in each alkyl moiety, a tetrahydropyranyl group, a 2,2-dimethyl-3-(2,2-dichlorovinyl)cyclopropylcarbonyl group, a formyl group, a C(2—5) alkanoyl group, a C(2—5) haloalkanoyl group containing up to 3 halogen atoms, or an optionally substituted phenyl or benzoyl group in which the optional substituents are selected from halogen atoms and C(1—4) alkyl, trifluoromethyl, cyano, nitro and dimethylaminophenylazo groups.

7. A composition as claimed in claim 6, in which R represents a hydrogen atom; an alkoxyalkyl group having 1 or 2 carbon atoms in each alkyl moiety; a formyl group; a benzoyl group; or a dimethylaminophenylazobenzoyl group.

8. A composition as claimed in any one of claims 1 to 7 which comprises at least two carriers, at least one of which is a surface-active agent.

9. A process for the preparation of an N-substituted 2-(nitromethylene)-tetrahydro-2H-1,3-thiazine as defined in claim 1, which comprises reacting the compound of formula:

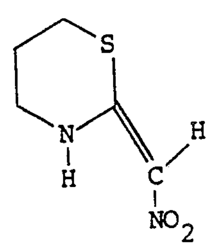

(II)

with a sulphonyl halide of formula

$$R—X—A—SO_2—Hal$$

in which R, X and A are as defined above, and Hal represents a halogen atom, in the presence of a base, to produce a compound of the general formula I in which n is 0; and if desired, converting the resulting

compound of the general formula I into any other desired compound of the general formula I.

10. A process for the preparation of an N-substituted 2-(nitromethylene)-tetrahydro-2H-1,3-thiazine as defined in claim 1 in which X represents an oxygen or sulphur atom and R represents a formyl, alkanoyl, haloalkanoyl, optionally substituted cycloalkanoyl or optionally substituted benzoyl group, which comprises reacting a compound of the general formula

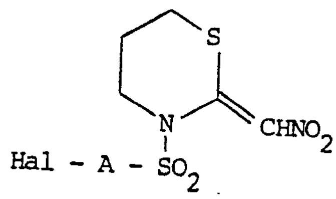

(III)

in which Hal represents a halogen atom and A has the meaning given in claim 1, with an acid of the general formula

R X H

or a salt thereof, in which R and X have the meanings given above.

11. A method of combating pests at a locus, which comprises applying to the locus a composition as claimed in any one of claims 1 to 8.

**Patentansprüche für die Vertragsstaaten: BE CH DE FR GB IT LI LU NL SE**

1. N-substituiertes 2-Nitromethylen-tetrahydro-2H-1,3-thiazin der allgemeinen Formel:

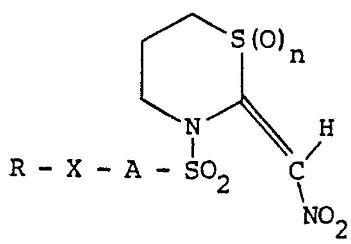

(I)

worin n den Wert 0 oder 1 aufweist; A eine Alkylengruppe mit 1 bis 10 Kohlenstoffatomen bedeutet; X ein Sauerstoff- oder Schwefelatom oder eine Gruppe —NR[1]— bedeutet, worin R[1] ein Wasserstoffatom oder eine Alkylgruppe mit bis zu 6 Kohlenstoffatomen darstellt oder, zusammen mit R, eine Alkylengruppe mit 4 bis 6 Kohlenstoffatomen bedeutet; und R ein Wasserstoffatom; eine Alkyl-, Halogenalkyl-, Alkoxyalkyl-, Tetrahydropyranyl-, Alkylcarbamoyl-, Dialkylcarbamoyl-, Formyl-, Alkanoyl- oder Halogenalkanoylgruppe, worin ein Alkylrest bis zu 6 Kohlenstoffatome aufweist; eine Cycloalkanoylgruppe mit 3 bis 6 Ringkohlenstoffatomen, die gegebenenfalls durch ein oder mehrere Halogenatome und/oder Alkylgruppen mit bis zu 6 Kohlenstoffatomen, Halogenalkylgruppen mit bis zu 6 Kohlenstoffatomen oder Dichlorvinylgruppen substituiert ist; oder eine Phenylgruppe oder Benzoylgruppe bedeutet, die gegebenenfalls durch Substituenten, ausgewählt unter Halogenatomen und Alkyl-, Alkoxy-, Halogenalkyl-, Halogenalkoxy-, Cyano-, Nitro-, Amino-, Hydroxy-, Dimethylaminophenylazo- und Alkoxycarbonylgruppen substituiert sind, worin ein Alkylrest bis zu 6 Kohlenstoffatome aufweist; mit der Maßgabe, daß dann, wenn X Sauerstoff bedeutet, R nicht für Alkyl, Alkanoyl oder gegebenenfalls substituiertes Phenyl steht.

2. Verbindung nach Anspruch 1, worin A eine geradkettige Alkylengruppe mit 3, 4 oder 5 Kohlenstoffatomen bedeutet.

3. Verbindung nach Anspruch 1 oder 2, worin R[1] eine C(1—4)alkylgruppe oder ein Wasserstoffatom bedeutet.

4. Verbindung nach Anspruch 1 oder 2, worin X ein Sauerstoffatom bedeutet.

5. Verbindung nach einem der Ansprüche 1 bis 4, worin n den Wert 0 aufweist.

6. Verbindung nach einem der Ansprüche 1 bis 5, worin R ein Wasserstoffatom, ein C(1—6)alkylgruppe, eine C(1—6)halogenalkylgruppe mit bis zu 3 Halogenatomen, eine Alkoxyalkylgruppe mit 1 bis 4 Kohlenstoffatomen in jedem Alkylrest, eine Tetrahydropyranylgruppe, eine 2,2-Dimethyl-3-(2,2-dichlorvinyl)cyclopropylcarbonylgruppe, eine Formylgruppe, eine C(2—5)alkanoylgruppe, eine C(2—5)halogenalkanoylgruppe mit bis zu 3 Halogenatomen, oder eine gegebenenfalls substituierte Phenyl- oder Benzoylgruppe bedeutet, worin die fakultativen Substituenten ausgewählt sind unter Halogenatomen und C(1—4)alkylgruppen, Trifluormethylgruppen, Cyanogruppen, Nitrogruppen und Dimethylaminophenylazogruppen.

7. Verbindung nach Anspruch 6, worin R ein Wasserstoffatom; eine Alkoxyalkylgruppe mit 1 oder 2

Kohlenstoffatomen in jedem Alkylrest; eine Formylgruppe; eine Benzoylgruppe; oder eine Dimethylaminophenylazobenzoylgruppe bedeutet.

8. Verfahren zur Herstellung eines N-substituierten 2-(Nitromethylen)-tetrahydro-2H-1,3-thiazins, wie in Anspruch 1 beansprucht, welches ein Umsetzen der Verbindung der Formel:

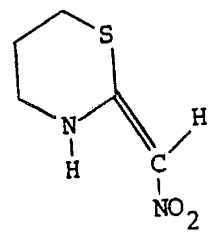

(II)

mit einem Sulfonylhalogenid der Formel

$$R—X—A—SO_2—Hal,$$

worin R, X und A wie vorstehend definiert sind und Hal ein Halogenatom bedeutet, in Gegenwart einer Base zur Ausbildung einer Verbindung der allgemeinen Formel I, worin n den Wert O hat; und, falls gewünscht, ein Umwandeln der erhaltenen Verbindung der allgemeinen Formel I in jede andere erwünschte Verbindung der allgemeinen Formel I umfaßt.

9. Verfahren zur Herstellung einer Verbindung, wie in Anspruch 1 beansprucht, worin X ein Sauerstoff- oder Schwefelatom darstellt und R eine Formylgruppe, Alkanoylgruppe, Halogenalkanoylgruppe, gegebenenfalls substituierte Cycloalkanoylgruppe oder eine gegebenenfalls substituierte Benzoylgruppe darstellt, welches ein Umsetzen einer Verbindung der allgemeinen Formel

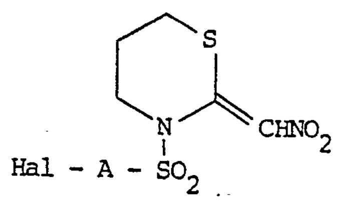

(III)

worin Hal ein Halogenatom bedeutet und A die für die allgemeine Formel I angeführte Bedeutung hat, mit einer Säure der allgemeinen Formel

R X H

oder einem Salz, insbesondere einem Alkalimetallsalz, hievon, in welcher Formel R und X die vorstehend angegebenen Bedeutungen besitzen, umfaßt.

10. Pestizide Zusammensetzung, welche eine Verbindung, wie in einem der Ansprüche 1 bis 7 beansprucht, zusammen mit einem Träger umfaßt.

11. Zusammensetzung nach Anspruch 10, welche wenigstens zwei Träger umfaßt, von denen wenigstens einer ein oberflächenaktives Mittel ist.

12. Verfahren zur Bekämpfung von Schädlingen an einem Ort, welches ein Aufbringen einer pestizid wirksamen Menge einer Verbindung, wie in einem der Ansprüche 1 bis 7 beansprucht, oder einer Zusammensetzung, wie in Anspruch 10 oder Anspruch 11 beansprucht, auf den Ort umfaßt.

**Patentansprüche für den Vertragsstaat: AT**

1. Pestizide Zusamensetzung, welche einen Träger und als wirksamen Bestandteil ein N-substituiertes 2-Nitromethylen-tetrahydro-2H-1,3-thiazin der allgemeinen Formel:

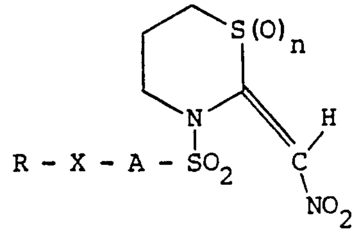

(I)

umfaßt, worin n den Wert 0 oder 1 aufweist; A eine Alkylengruppe mit 1 bis 10 Kohlenstoffatomen

bedeutet; X ein Sauerstoff- oder Schwefelatom oder eine Gruppe —$NR^1$— bedeutet, worin $R^1$ ein Wasserstoffatom oder eine Alkylgruppe mit bis zu 6 Kohlenstoffatomen darstellt oder, zusammen mit R, eine Alkylengruppe mit 4 bis 6 Kohlenstoffatomen bedeutet; und R ein Wasserstoffatom; eine Alkyl-, Halogenalkyl-, Alkoxyalkyl-, Tetrahydropyranyl-, Alkylcarbamoyl-, Dialkylcarbamoyl-, Formyl-, Alkanoyl- oder Halogenalkanoylgruppe, worin ein Alkylrest bis zu 6 Kohlenstoffatome aufweist; eine Cycloalkanoylgruppe mit 3 bis 6 Ringkohlenstoffatomen, die gegebenenfalls durch ein oder mehrere Halogenatome und/oder Alkylgruppen mit bis zu 6 Kohlenstoffatomen, Halogenalkylgruppen mit bis zu 6 Kohlenstoffatomen oder Dichlorvinylgruppen substituiert ist; oder eine Phenylgruppe oder Benzoylgruppe bedeutet, die gegebenenfalls durch Substituenten, ausgewählt unter Halogenatomen und Alkyl-, Alkoxy-, Halogenalkyl-, Halogenalkoxy-, Cyano-, Nitro-, Amino-, Hydroxy-, Dimethylaminophenylazo- und Alkoxycarbonylgruppen substituiert sind, worin ein Alkylrest bis zu 6 Kohlenstoffatome aufweist; mit der Maßgabe, daß dann, wenn X Sauerstoff bedeutet, R nicht für Alkyl, Alkanoyl oder gegebenenfalls substituiertes Phenyl steht.

2. Zusammensetzung nach Anspruch 1, worin A eine geradkettige Alkylengruppe mit 3, 4 oder 5 Kohlenstoffatomen bedeutet.

3. Zusammensetzung nach Anspruch 1 oder 2, worin $R^1$ eine C(1—4)alkylgruppe oder ein Wasserstoffatom bedeutet.

4. Zusammensetzung nach Anspruch 1 oder 2, worin X ein Sauerstoffatom bedeutet.

5. Zusammensetzung nach einem der Ansprüche 1 bis 4, worin n den Wert 0 aufweist.

6. Zusammensetzung nach einem der Ansprüche 1 bis 5, worin R ein Wasserstoffatom, ein C(1—6)alkylgruppe, eine C(1—6)halogenalkylgruppe mit bis zu 3 Halogenatomen, eine Alkoxyalkylgruppe mit 1 bis 4 Kohlenstoffatomen in jedem Alkylrest, eine Tetrahydropyranylgruppe, eine 2,2-Dimethyl-3-(2,2-dichlorvinyl)cyclopropylcarbonylgruppe, eine Formylgruppe, eine C(2—5)alkanoylgruppe, eine C(2—5)halogenalkanoylgruppe mit bis zu 3 Halogenatomen oder eine gegebenenfalls substituierte Phenyl- oder Benzoylgruppe bedeutet, worin die fakultativen Substituenten ausgewählt sind unter Halogenatomen und C(1—4)alkylgruppen, Trifluormethylgruppen, Cyanogruppen, Nitrogruppen und Dimethylaminophenylazogruppen.

7. Zusammensetzung nach Anspruch 6, worin R ein Wasserstoffatom; eine Alkoxyalkylgruppe mit 1 oder 2 Kohlenstoffatomen in jedem Alkylrest; eine Formylgruppe; eine Benzoylgruppe; oder eine Dimethylaminophenylazobenzoylgruppe bedeutet.

8. Zusammensetzung nach einem der Ansprüche 1 bis 7, welche wenigstens zwei Träger umfaßt, von denen wenigstens einer ein oberflächenaktives Mittel ist.

9. Verfahren zur Herstellung eines N-substituierten 2-(Nitromethylen)-tetrahydro-2H-1,3-thiazins, wie in Anspruch 1 definiert, welches ein Umsetzen der Verbindung der Formel:

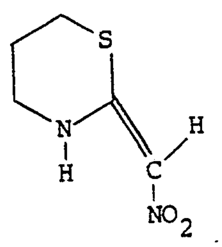

(II)

mit einem Sulfonylhalogenid der Formel

$$R—X—A—SO_2—Hal,$$

worin R, X und A wie vorstehend definiert sind und Hal ein Halogenatom bedeutet, in Gegenwart einer Base zur Ausbildung einer Verbindung der allgemeinen Formel I, worin n den Wert O hat; und, falls gewünscht, ein Umwandeln der erhaltenen Verbindung der allgemeinen Formel I in jede andere erwünschte Verbindung der allgemeinen Formel I umfaßt.

10. Verfahren zur Herstellung eines N-substituierten 2-(Nitromethylen)-tetrahydro-2H-1,3-thiazins, wie in Anspruch 1 definiert, worin X ein Sauerstoff- oder Schwefelatom darstellt und R eine Formylgruppe, Alkanoylgruppe, Halogenalkanoylgruppe, gegebenenfalls substituierte Cycloalkanoylgruppe oder gegebenenfalls substituierte Benzoylgruppe darstellt, welches ein Umsetzen einer Verbindung der allgemeinen Formel

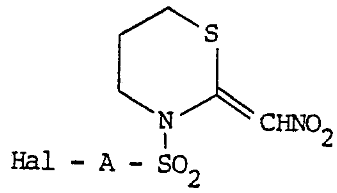

(III)

worin Hal ein Halogenatom bedeutet und A die für die allgemeine Formel I angeführte Bedeutung hat, mit einer Säure der allgemeinen Formel

R X H

oder einem Salz, insbesondere einem Alkalimetallsalz, hievon, in welcher Formel R und X die vorstehend angegebenen Bedeutungen besitzen, umfaßt.

11. Verfahren zur Bekämpfung von Schädlingen an einem Ort, welches ein Aufbringen einer Zusammensetzung, wie in einem der Ansprüche 1 bis 8 beansprucht, auf den Ort umfaßt.

**Revendications pour les Etats contractants: BE CH DE FR GB IT LI LU NL SE**

1. 2-nitrométhylène-tétrahydro-2H-1,3-thiazine N-substituée de formule générale:

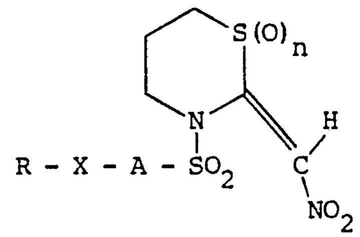

(I)

où n représente 0 ou 1; A représente un groupe alcoylène ayant de 1 à 10 atomes de carbone; X représente un atome d'oxygène ou de soufre ou un groupe —$NR^1$— où $R^1$ représente un atome d'hydrogène ou un groupe alcoyle ayant jusqu'à 6 atomes de carbone ou, avec R, représente un groupe alcoyle ayant de 4 à 6 atomes de carbone, et R représente un atome d'hydrogène; un groupe alcoyle, haloalcoyle, alcoxyalcoyle, tétrahydropyranyle, alcoylcarbamoyle, dialcoylcarbamoyle, formyle, alcanoyle ou haloalcanoyle, où toute fraction alcoyle a jusqu'à 6 atomes de carbone; un groupe cycloalcanoyle ayant de 3 à 6 atomes de carbone dans son noyau éventuellement substitué par un ou plusieurs atomes d'halogène et/ou des groupes alcoyle ayant jusqu'à 6 atomes de carbone, des groupes haloalcoyle ayant jusqu'à 6 atomes de carbone ou des groupes dichlorovinyle; ou un groupe phényle ou benzoyle éventuellement substitué par des substituants choisis entre les atomes d'halogène et les groupes alcoyle, alcoxy, haloalcoyle, haloalcoxy, cyano, nitro, amino, hydroxy, diméthylaminophénylazo et alcoxycarbonyle, où toute fraction alcoyle a jusqu'à 6 atomes de carbone; à condition que si X est un oxygène alors R n'est pas un alcoyle, un alcanoyle ou un phényle éventuellement substitué.

2. Composé selon la revendication 1, où A représente un groupe alcoylène à chaîne droite ayant 3, 4 ou 5 atomes de carbone.

3. Composé selon la revendication 1 ou la revendication 2, où $R^1$ représente un groupe alcoyle en $C_1$ à $C_4$ ou un atome d'hydrogène.

4. Composé selon la revendication 1 ou la revendication 2, où X représente un atome d'oxygène.

5. Composé selon l'une quelconque des revendications 1 à 4, où n vaut 0.

6. Composé selon l'une quelconque des revendications 1 à 5, où R représente un atome d'hydrogène, un groupe alcoyle en $C_1$ à $C_6$, un groupe haloalcoyle en $C_1$ à $C_6$ contenant jusqu'à 3 atomes d'halogène, un groupe alcoxyalcoyle ayant de 1 à 4 atomes de carbone dans chaque fraction alcoyle, un groupe tétrahydropyranyle, un groupe 2,2-diméthyl-3-(2,2-dichlorovinyl)cyclopropylcarbonyle, un groupe formyle, un groupe alcanoyle en $C_2$ à $C_5$, un groupe haloalcanoyle en $C_2$ à $C_5$ contenant jusqu'à 3 atomes d'halogène, ou un groupe phényle ou benzoyle éventuellement substitué dans lequel les substituants éventuels sont choisis entre les atomes d'halogène et les groupes alcoyle en $C_1$ à $C_4$, trifluorométhyle, cyano, nitro et diméthylaminophénylazo.

7. Composé selon la revendication 6, où R représente un atome d'hydrogène; un groupe alcoxyalcoyle ayant 1 ou 2 atomes de carbone dans chaque fraction alcoyle; un groupe formyle; un groupe benzoyle; ou un groupe diméthylaminophénylazobenzoyle.

8. Procédé de préparation d'une 2-(nitrométhylène)-tétrahydro-2H-1,3-thiazine N-substituée selon la revendication 1, dans lequel on fait réagir le composé de formule:

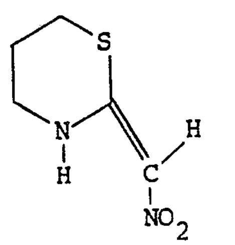

(II)

avec un halogénure de sulfonyle de formule

$$R—X—A—SO_2—Hal$$

où R, X et A sont tels que définis ci-dessus et Hal représente un atome d'halogène, en présence d'une base, pour produire un composé de formule générale I où n vaut O; et si on le désire, on transforme le composé résultant de formule générale I en n'importe quel autre composé désiré de formule générale I.

9. Procédé de préparation d'un composé selon la revendication 1 où X représente un atome d'oxygène ou de soufre et R représente un groupe formyle, alcoanoyle, haloalcanoyle, cycloalcanoyle éventuellement substitué ou benzoyle éventuellement substitué, dans lequel on fait réagir un composé de formule générale

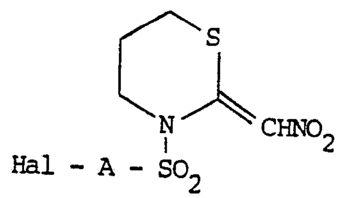

(III)

où Hal représente un atome d'halogène et A a la signification donnée dans la revendication 1, avec un acide de formule générale

R X H

ou un de ses sels, où R et X ont la signification donnée ci-dessus.

10. Composition pesticide comprenant un composé selon l'une quelconque des revendications 1 à 7, avec un support.

11. Composition selon la revendication 10, comprenant au moins deux supports, dont au moins un est un agent tensio-actif.

12. Procédé pour combattre les parasites en un lieu, dans lequel on applique en ce lieu une quantité efficace comme pesticide d'un composé selon l'une quelconque des revendications 1 à 7 ou d'une composition selon la revendication 10 ou la revendication 11.

**Revendications pour l'Etat contractant: AT**

1. Composition pesticide qui comprend un support et, comme ingrédient actif, une 2-nitrométhylène-:étrahydro-2H-1,3-thiazine N-substituée de formule générale:

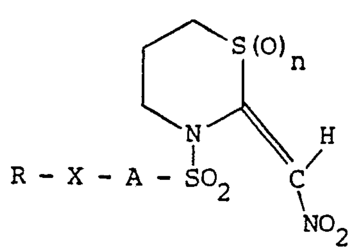

(I)

où n représente 0 ou 1; A représente un groupe alcoylène ayant de 1 à 10 atomes de carbone; X représente un atome d'oxygène ou de soufre ou un groupe —$NR^1$— où $R^1$ représente un atome d'hydrogène ou un groupe alcoyle ayant jusqu'à 6 atomes de carbone ou, avec R, représente un groupe alcoyle ayant de 4 à 6 atomes de carbone, et R représente un atome d'hydrogène; un groupe alcoyle, haloalcoyle, alcoxyalcoyle, tétrahydropyranyle, alcoylcarbamoyle, dialcoylcarbamoyle, formyle, alcanoyle ou haloalcanoyle, où toute fraction alcoyle a jusqu'à 6 atomes de carbone; un groupe cycloalcanoyle ayant de 3 à 6 atomes de carbone dans son noyau éventuellement substitué par un ou plusieurs atomes d'halogène et/ou des groupes alcoyle ayant jusqu'à 6 atomes de carbone, des groupes haloalcoyle ayant jusqu'à 6 atomes de carbone ou des groupes dichlorovinyle; ou un groupe phényle ou benzoyle éventuellement substitué par des substituants choisis entre les atomes d'halogène et les groupes alcoyle, alcoxy, haloalcoyle, haloalcoxy, cyano, nitro, amino, hydroxy, diméthylaminophénylazo et alcoxycarbonyle, où toute fraction alcoyle a jusqu'à 6 atomes de carbone; à condition que si X est un oxygène alors R n'est pas un alcoyle, un alcanoyle ou un phényle éventuellement substitué.

2. Composition selon la revendication 1, où A représente un groupe alcoylène à chaîne droite ayant 3, 4 ou 5 atomes de carbone.

3. Composition selon la revendication 1 ou la revendication 2, où $R^1$ représente un groupe alcoyle en $C_1$ à $C_4$ ou un atome d'hydrogène.

4. Composition selon la revendication 1 ou la revendication 2, où X représente un atome d'oxygène.

5. Composition selon l'une quelconque des revendications 1 à 4, où n vaut 0.

6. Composition selon l'une quelconque des revendications 1 à 5, où R représente un atome d'hydrogène, un groupe alcoyle en $C_1$ à $C_6$, un groupe haloalcoyle en $C_1$ à $C_6$ contenant jusqu'à 3 atomes

d'halogène, un groupe alcoxyalcoyle ayant de 1 à 4 atomes de carbone dans chaque fraction alcoyle, un groupe tétrahydropyranyle, un groupe 2,2-diméthyl-3-(2,2-dichlorovinyl)cyclopropylcarbonyle, un groupe formyle, un groupe alcanoyle en $C_2$ à $C_5$, un groupe haloalcanoyle en $C_2$ à $C_5$ contenant jusqu'à 3 atomes d'halogène, ou un groupe phényle ou benzoyle éventuellement substitué dans lequel les substituants éventuels sont choisis entre les atomes d'halogène et les groupes alcoyle en $C_1$ à $C_4$, trifluorométhyle, cyano, nitro et diméthylaminophénylazo.

7. Composition selon la revendication 6, où R représente un atome d'hydrogène; un groupe alcoxyalcoyle ayant 1 ou 2 atomes de carbone dans chaque fraction alcoyle; un groupe formyle; un groupe benzoyle; ou un groupe diméthylaminophénylazobenzoyle.

8. Composition selon l'une quelconque des revendications 1 à 7 qui comprend au moins deux supports, dont au moins un est un agent tensio-actif.

9. Procédé de préparation d'une 2-(nitrométhylène)-tétrahydro-2H-1,3-thiazine N-substituée selon la revendication 1, dans lequel on fait réagir le composé de formule:

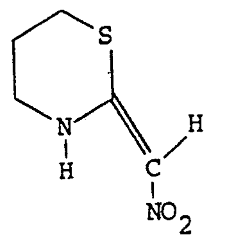

(II)

avec un halogénure de sulfonyle de formule

$$R—X—A—SO_2—Hal$$

où R, X et A sont tels que définis ci-dessus et Hal représente un atome d'halogène, en présence d'une base, pour produire un composé de formule générale I où n vaut O; et si on le désire, on transforme le composé résultant de formule générale I en n'importe quel autre composé désiré de formule générale I.

10. Procédé de préparation d'un 2-(nitrométhylène)-tétrahydro-2H-1,3-thiazine N-substituée selon la revendication 1 où X représente un atome d'oxygène ou de soufre et R représente un groupe formyle, alcoanoyle, haloalcanoyle, cycloalcanoyle éventuellement substitué ou benzoyle éventuellement substitué, dans lequel on fait réagir un composé de formule générale

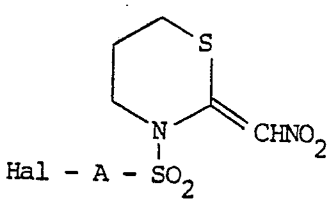

(III)

où Hal représente un atome d'halogène et A a la signification donnée dans la revendication 1, avec un acide de formule

$$R X H$$

ou un de ses sels, où R et X ont les significations données ci-dessus.

11. Procédé pour combattre les parasites en un lieu, dans lequel on applique à ce lieu une composition selon l'une quelconque des revendications 1 à 8.